(19) Europäisches Patentamt
European Patent Office
Office européen des brevets

(11) Numéro de publication: **0 021 940**
**B1**

(12) # FASCICULE DE BREVET EUROPEEN

(45) Date de publication du fascicule du brevet: **19.01.83**

(51) Int. Cl.[3]: **C 07 D 277/62**, C 07 D 277/64, A 61 K 31/425

(21) Numéro de dépôt: **80400831.6**

(22) Date de dépôt: **10.06.80**

(54) **Nouveaux dérivés aminés du benzothiazole, leur procédé de préparation et leur application en thérapeutique.**

(30) Priorité: **20.06.79 FR 7915774**

(43) Date de publication de la demande: **07.01.81 Bulletin 81/1**

(45) Mention de la délivrance du brevet: **19.01.83 Bulletin 83/3**

(84) Etats contractants désignés: **BE CH DE GB IT LI NL SE**

(56) Documents cités: **FR-A-2 406 635**

(73) Titulaire: **LABORATOIRES JACQUES LOGEAIS Société dite:, 71, Avenue du Général de Gaulle, F-92130 Issy-Les-Moulineaux (FR)**

(72) Inventeur: **Maillard, Jacques Georges, 82 bis Avenue de Paris, F-78000 Versailles (FR)**
Inventeur: **Delaunay, Pierre Paul André, 26 Rue des Grosses Eaux, F-95220 Herblay (FR)**
Inventeur: **Legeai, Jacky Marcel Gérard, 92 Avenue du Général Leclerc, F-91120 Palaiseau (FR)**

(74) Mandataire: **Polus, Camille et al, c/o Cabinet Lavoix 2, Place d'Estienne d'Orves, F-75441 Paris Cedex 09 (FR)**

**EP 0 021 940 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Nouveaux dérivés aminés du benzothiazole, leur procédé de préparation et leur application en thérapeutique

La présente invention concerne des dérivés aminés du benzothiazole, leur procédé de préparation et leur application en thérapeutique.

La Demanderesse a déjà décrit dans la demande de brevet FR-A-2 406 635 des dérivés aminés du 4,5,6,7-tétrahydro benzo[d]thiazole, qui présentent une activité intéressante sur le système nerveux central analogue à celle des stimulants dopaminergiques, qui s'accompagne d'une action cardiovasculaire biphasique (hypotension aux faibles doses, hypertension aux doses élevées).

La présente invention a pour objet de nouveaux dérivés aminés du 4,5,6,7-tétrahydro benzo[d]thiazole qui n'ont pu être préparés que par une voie de synthèse totalement différente et qui augmentent le débit sanguin périphérique sans provoquer d'hypertension et peuvent être utilisés en thérapeutique en raison de cette action.

Ainsi, la présente invention a pour objet des composés de formule générale

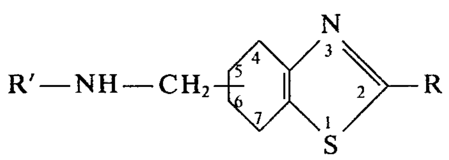

(I)

dans laquelle R représente un atome d'hydrogène ou un radical alcoyle en $C_1$ à $C_6$
R'représente un atome d'hydrogène ou un radical alcoyle en $C_1$ à $C_6$
ainsi que leurs sels d'addition aver des acides pharmaceutiquement acceptables.

Les sels d'addition peuvent être notamment ceux formés avec les acides chlorhydrique, bromhydrique, sulfurique et les acides organiques pharmaceutiquement acceptables.

La présente invention a également pour objet un procédé de préparation des composés de formule I qui est caractérisé en ce que

a) on fait réagir sur un céto-ester bromé de formule

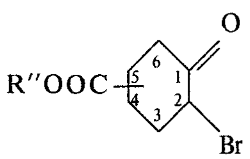

(II)

dans laquelle $R''$ est un radical alcoyle en $C_1 - C_2$, un thioamide de formule

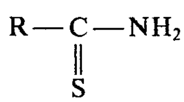

dans laquelle R a la signification donnée précédemment, obtenant ainsi un ester de formule

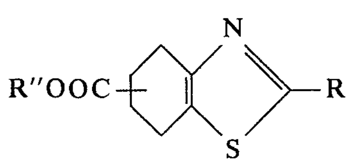

(III)

b) on réduit l'ester de formule III à l'aide d'aluminohydrure de lithium en un alcool de formule

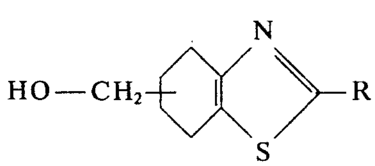

(IV)

c) on transforme l'alcool de formule IV au moyen de chlorure de p-toluènesulfonyle en le tosylate correspondant de formule

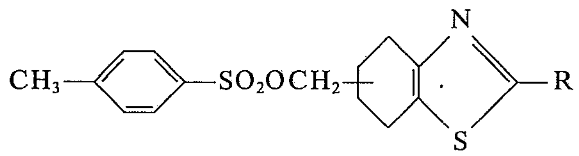

(V)

d) on fait réagir sur le tosylate de formule V une amine de formule $R'-NH_2$, R' ayant la signification donnée ci-dessus de façon à obtenir une amine de formule I, et éventuellement
e) on transforme l'amine de formule I en un sel avec un acide pharmaceutiquement acceptable.

Certains des céto-esters bromés (II) utilisés comme produits de départ sont connus:

bromo-2 éthoxycarbonyl-6 cyclohexanone (J. E. BRENNER J. Org. chem. 1961, 26, 22)
bromo-2 éthoxycarbonyl-4 cyclohexanone (L. BERGER et Coll. DE-2 214 501, 5 oct. 1972, Chem. Abstr. 1973, 78, 29609b).

Les autres céto-esters bromés de formule II sont préparés de manière analogue.

La réaction avec un thioamide peut être effectuée au sein d'un solvant tel qu'un alcool (éthanol, isopropanol) ou le diméthyl-formamide, à température ordinaire pour les thioamides de faible poids moléculaire ($R=H$, $CH_3$) ou en chauffant légèrement (40—60° C) pour les thioamides supérieurs.

Le benzothiazole (III) formé peut être isolé, après évaporation du solvant, grâce à son caractère faiblement basique (dissolution en milieu aqueux acide).

Le stade (b) qui consiste à réduire l'ester (III) en alcool (IV) au moyen de l'aluminohydrure de lithium peut être effectué dans l'éther ou le tétrahydrofuranne, à température ordinaire (ou, de préférence, à basse température $<0°$ C, pour les dérivés dans lesquels $R=H$).

Les alcools (IV) peuvent être isolés et purifiés par les moyens habituels (distillation, cristallisation) ou directement transformés en tosylates (V) au moyen du chlorure de p. toluènesulfonyle, en présence d'une base tertiaire telle que la pyridine.

Les tosylates (V) peuvent être purifiés par cristallisation ou utilisés bruts pour la réaction avec l'amine $R'NH_2$. Pour cette dernière réaction, on utilise avantageusement un excès d'amine primaire $R'NH_2$ au sein d'un solvant tel que le méthanol ou l'éthanol en autoclave et à température voisine de 100° C.

Les amines (I) formées sont obtenues par évaporation du solvant et distillées sous pression réduite. Ce sont des bases fortes, qui se carbonatent assez facilement à l'air libre. Elles sont avantageusement transformées en sels, tels que les chlorhydrates, par addition d'un léger défaut d'acide anhydre, dans un solvant inerte tel que l'éther.

Les exemples suivants illustrent la présente invention.

## Exemple 1

### Aminométhyl-4 tétrahydro-4,5,6,7benzo[d]thiazole, et son chlorhydrate
### ($I$, $R=R'=H$, substitution en 4)

#### 1) Ethoxycarbonyl-4 tétrahydro-4,5,6,7 benzo[d]thiazole (III)

90 g (0,36 mole) de bromo-2 éthoxycarbonyl-6 cyclohexanone (II) sont dissous dans 300 ml d'éthanol anhydre contenant 22 g (0,36 mole) de thioformamide. Après 60 h à température ordinaire et évaporation du solvant, le résidu est repris par de l'oxyde d'isopropyle et extrait plusieurs fois par HCL 2N. La solution acide est alcalinisée et l'huile libérée est extraite à l'éther, puis distillée. $eb_{0,1}=100-104°$ C. Rendt: 31%.

#### 2) Hydroxyméthyl-4 tétrahydro-4,5,6,7 benzo[d]thiazole (IV)

A une solution de 1,14 g (30 m.moles) de $AlLiH_4$ dans 50 ml de tétrahydrofuranne anhydre, refroidie et maintenue à $-20°$ C, on ajoute lentement 6,66 g (30 m.moles) du dérivé (III) précédent dissous dans 30 ml de THF anhydre. Après 30 minutes à $-20°$ C, l'excès de $AlLiH_4$ est détruit par addition d'isopropanol à $-20°$ C, puis de 5 ml de solution aqueuse saturée de chlorure de sodium, et agitation pendant 2 heures. Le précipité formé est filtré et lavé à l'acétate d'éthyle, le filtrat est évaporé et le

résidu huileux est repris à chaud par de l'hexane; le produit (IV) cristallise par refroidissement Rendement 70%—F: 74—75° C.

3) Tosyloxyméthyl-4 tétrahydro-4,5,6,7 benzo[d]thiazole (V)

9,95 g (52 m.moles) de chlorure de p.toluènesulfonyle sont ajoutés par portions à un mélange de 8 g (47 m.moles) du dérivé (IV) précédent et 15 g de pyridine anhydre, en maintenant la température à 10° C. Après 24 h d'agitation à température ordinaire, le mélange est hydrolysé et extrait par l'acétate d'éthyle. Les phases organiques réunies sont lavées à l'eau, séchées et évaporées. L'huile résiduelle cristallise lentement. Le produit est recristallisé dans de l'oxyde d'isopropyle bouillant, par refroidissement. Rendement 72%—F: 64—65° C.

4) Aminométhyl-4 tétrahydro-4,5,6,7 benzo[d]thiazole et chlorhydrate (I, R = R' = H)

Une solution de 26 g (80 m.moles) du dérivé (V) précédent dans 40 ml de méthanol est chauffée avec 180 g d'ammoniac, en autoclave, pendant 5 h à 90° C. Après évaporation du solvant, et addition de 3,8 g (96 m.moles) de soude en solution dans 25 ml d'eau, l'amine formée est extraite plusieurs fois à l'éther, séchée et distillée. $eb_{0,1}$ = 97—100° C. Rendement 54%.

L'amine (I, R = R' = H) est transformée en chlorhydrate par addition d'un léger défaut d'HCl anhydre à une solution de la base dans l'éther. Rendement: 98% —F: 210° C.

Exemple 2

(N-méthylaminométhyl)-4 tétrahydro-4,5,6,7 benzo[d]thiazole et son chlorhydrate
(I, R = H, R' = $CH_3$, substitution en 4)

30,4 g (93 m.moles) du dérivé (V) décrit dans l'exemple 1 sont agités pendant 60 h, à température ordinaire, avec une solution de 150 g de méthylamine dans 150 ml d'eau. Le mélange est homogène après 48 h. La solution est évaporée à sec, le résidu est repris par un excès de soude 2N et extrait au chlorure de méthylène. Le résidu huileux d'évaporation du solvant est distillé. $eb_{0,2}$: 96—99° C. Rendement 80%.

L'amine est transformée en chlorhydrate par addition d'un léger défaut d'HCl anhydre, dans l'éther. Rendement 88%—F: 120° C (avec décomp.).

Exemple 3

Méthyl-2 aminométhyl-4 tétrahydro-4,5,6,7 benzo[d]thiazole et son chlorhydrate
(I, R = $CH_3$, R' = H, substitution en 4)

1) Méthyl-2 éthoxycarbonyl-4 tétrahydro-4,5,6,7 benzo[d]thiazole (III)

Il est préparé par action de thioacétamide sur la bromo-2 carbéthoxy-6 cyclohexanone (II), selon le procédé décrit dans l'exemple 1. $eb_{0,1}$ = 102—105° C.

2) Méthyl-2 hydroxyméthyl-4 tétrahydro-4,5,6,7 benzo[d]thiazole (IV)

Il est préparé par réduction du dérivé (III) précédent au moyen de $AlLiH_4$ selon le procédé décrit dans l'exemple 1. $eb_{0,4}$ = 102—106° C. Rendement 82%.

3) Méthyl-2 tosyloxyméthyl-4 tétrahydro-4,5,6,7 benzo[d]thiazole (V)

Il est préparé par action du chlorure de p. toluène sulfonyle sur l'alcool (IV) précédent, selon le procédé décrit dans l'exemple 1. F = 97 – 98° C — Rdt 89%.

4) Méthyl-2 aminométhyl-4 tétrahydro-4,5,6,7 benzo[d]thiazole et son chlorhydrate
(I, R = $CH_3$, R' = H)

Il est préparé par action de l'ammoniac sur le tosylate (V) ci-dessus, selon le procédé décrit dans

l'exemple 1. $eb_{0,1} = 76-78°C$. Rendement: 75%.
(base se carbonatant rapidement à l'air).

L'amine est transformée en chlorhydrate par HCl anhydre dans l'éther. F = 140° C (avec décomp.) Rendement: 90%.

## Exemple 4

### Méthyl-2 (N-méthylaminométhyl)-4 tétrahydro-4,5,6,7 benzo[d]thiazole et son chlorhydrate (I, R = R' = $CH_3$, substitution en 4)

Il est préparé à partir du tosylate (V) décrit dans l'exemple 3, par action d'un large excès de méthylamine en solution dans le méthanol, à 130° C en autoclave. L'amine est isolée par le procédé habituel, décrit dans les exemples précédents. $eb_{0,5} = 98-102°C$ — Rdt: 38%.

L'amine est transformée en chlorhydrate par un léger défaut d'HCl anhydre, en solution dans l'éther. F = 144° C (avec décom.) — Rendement: 75%.

## Exemple 5

### (N-méthylaminométhyl)-5 tétrahydro-4,5,6,7 benzo[d]thiazole et son chlorhydrate (I, R = H, R' = $CH_3$, substitution en 5)

#### 1) Méthoxycarbonyl-5 tétrahydro-4,5,6,7 benzo[d]thiazole (III)

Il est préparé par action du thioformamide sur la bromo-2 méthoxycarbonyl-5 cyclohexanone, selon le procédé décrit dans l'exemple 1. $eb_{0,4} = 110-120°C$ — Rendement: 35,5%.

#### 2) Hydroxyméthyl-5 tétrahydro-4,5,6,7 benzo[d]thiazole (IV)

Il est préparé par réduction du compose (III) précédent par $AlLiH_4$, selon le procédé décrit dans l'exemple 1. $eb_{0,6} = 138-144°C$ — Rendement: 81%.

#### 3) Tosyloxyméthyl-5 tétrahydro-4,5,6,7 benzo[d]thiazole (V)

Il est préparé à partir du dérivé (IV) précédent selon le procédé décrit dans l'exemple 1. F = 87 – 88° C — Rendement: 70%.

#### 4) (N-méthylaminométhyl)-5 tétrahydro-4,5,6,7 benzo[d]thiazole et son chlorhydrate (I, R = H, R' = $CH_3$)

Il est préparé par action de la méthylamine en solution dans l'éthanol, sur le tosylate (IV) précédent en autoclave à 100° C. $eb_{0,5} = 115-120°C$ — Rdt: 82%.
Chlorhydrate, F: 201—202° C — Rendt: 82%.

## Exemple 6

### Méthyl-2 (N-méthylaminométhyl)-5 tétrahydro-4,5,6,7 benzo[d]thiazole et son chlorhydrate (I, R = R' = $CH_3$, substitution en 5)

#### 1) Méthyl-2 éthoxycarbonyl-5 tétrahydro-4,5,6,7 benzo[d]thiazole (III)

Il est préparé par action du thioacétamide sur la bromo-2 éthoxycarbonyl-5 cyclohexanone (II), selon le procédé décrit dans l'exemple 1. $b_{0,4} = 116-120°C$.

#### 2) Méthyl-2 hydroxyméthyl-5 tétrahydro-4,5,6,7 benzo[d]thiazole (IV)

Il est préparé par réduction du dérivé (III) précédent au moyen de $AlLiH_4$, selon le procédé décrit dans l'exemple 1. $eb_{0,5} = 120-140°C$.

Le produit brut est purifié par cristallisation dans le tétrachlorure de carbone. F: 52—55° C.

3) Méthyl-2 tosyloxyméthyl-5 tétrahydro-4,5,6,7 benzo[d]thiazole (V)

Il est préparé par action du chlorure de p. toluène sulfonyle sur l'alcool (IV) précédent selon le procédé décrit dans l'exemple 1. F = 105—106° C — Rdt 74%.

4) Méthyl-2 (N-méthylaminométhyl)-5 tétrahydro-4,5,6,7 benzo[d]thiazole (I, R = R' = $CH_3$)

Il est préparé par action de la méthylamine sur le tosylate (V) ci-dessus, en solution dans le méthanol, en autoclave à 100° C. $eb_{0,5}$ = 100—110° C — Rendement: 75%.

L'amine est transformée en chlorhydrate selon le procédé décrit dans l'exemple 1. F = 248—250° C (decomp.) — Rendement: 80%.

Exemple 7

Méthyl-2 (N-méthylaminométhyl)-6 tétrahydro-4,5,6,7 benzo[d]thiazole et son chlorhydrate
(I, R = R' = $CH_3$, substitution en 6)

1) Méthyl-2 éthoxycarbonyl-6 tétrahydro-4,5,6,7 benzo[d]thiazole (III)

Il est préparé par action du thioacétamide sur la bromo-2 éthoxycarbonyl-4 cyclohexanone selon le procédé décrit dans l'exemple 1. $eb_{0,3}$ = 110—120° C — Rendement: 30%.

2) Méthyl-2 hydroxyméthyl-6 tétrahydro-4,5,6,7 benzo[d]thiazole (IV)

Il est préparé par réduction du dérivé (III) précédent selon le procédé décrit dans l'exemple 1. $eb_{0,4}$ = 145—155° C. F = 101—102° C — Rendement: 70%.

3) Méthyl-2 tosyloxyméthyl-6 tétrahydro-4,5,6,7 benzo[d]thiazole (V)

Il est préparé par action du chlorure de p. toluène sulfonyle sur le dérivé (IV) précédent, selon le procédé décrit dans l'exemple 1. F: 70—71° C — Rendement: 71%.

4) Méthyl-2 (N-méthylaminométhyl)-6 tétrahydro-4,5,6,7 benzo[d]thiazole et son chlorhydrate
(I, R = R' = $CH_3$)

Il est préparé par action de la méthylamine en solution dans le méthanol, sur le tosylate (V) précédent, en autoclave à 110° C. $eb_{0,3}$ = 105—115° C — Rdt 83%.

La base est transformée en chlorhydrate par un léger défaut d'HCl dans l'éther anhydre. F. = 176—178° C — Rendement: 93%.

Exemple 8

Aminométhyl-6 tétrahydro-4,5,6,7 benzo[d]thiazole et son chlorhydrate
(I, R = R' = H, substitution en 6)

1) Ethoxycarbonyl-6 tétrahydro-4,5,6,7 benzo[d]thiazole (III)

Il est obtenu par action du thioformamide sur la bromo-2 éthoxycarbonyl-4 cyclohexanone, selon le procédé décrit dans l'exemple 1. $eb_{0,2}$ = 105—115° C — Rendement: 27%.

2) Hydroxyméthyl-6 tétrahydro-4,5,6,7 benzo[d]thiazole (IV)

Il est préparé par réduction du dérivé (III) précédent selon le procédé décrit dans l'exemple 1. $eb_1$ = 140—150° C — Rendement: 66%.

3) Tosyloxyméthyl-6 tétrahydro-4,5,6,7 benzo[d]thiazole (V)

Il est préparé à partir du dérivé (IV) précédent selon le procédé décrit dans l'exemple 1. F: 90—91°C — Rendement: 64%.

4) Aminométhyl-6 tétrahydro-4,5,6,7 benzo[d]thiazole (I, R=R'=H)

Il est préparé par action de l'ammoniac sur le tosylate V précédent, dans le méthanol, à l'autoclave à 100°C. $eb_{0,5}$=100—106°C — Rendement: 42%. Chlorhydrate, F: 218—219°C — Rendement: 95%.

Exemple 9

Ethyl-2 (N-éthylaminométhyl)-6 tétrahydro-4,5,6,7 benzo[d]thiazole et son chlorhydrate
(I, R=R'=$C_2H_5$, substitution en 6)

1) Ethoxycarbonyl-6 tétrahydro-4,5,6,7 benzo[d]thiazole (III)

Il est prépare par action du thiopropionamide sur la bromo-2 éthoxycarbonyl-4 cyclohexanone dans le diméthylformamide à température ordinaire. $eb_{0,2}$=112—118°C — Rendement: 42%.

2) Ethyl-2 hydroxyméthyl-6 tétrahydro-4,5,6,7 benzo[d]thiazole (IV)

Il est préparé par réduction du dérivé III précédent par $AlLiH_4$ dans l'éther à température ordinaire. $eb_{0,5}$=142—144°C — Rendement: 86%.

3) Ethyl-2 tosyloxyméthyl-6 tétrahydro-4,5,6,7 benzo[d]thiazole (V)

Il est préparé par action du chlorure de p. toluènesulfonyle sur le dérivé (IV) précédent, selon le procédé décrit dans l'exemple 1. F=54—56°C — Rdt 65%.

4) Ethyl-2 (N-éthyl-aminométhyl)-6 tétrahydro-4,5,6,7 benzo[d]thiazole et son chlorhydrate
(I, R=R'=$C_2H_5$)

Il est préparé par action de l'éthylamine en solution méthanolique sur le tosylate (V) précédent en autoclave à 90°C. $eb_{0,1}$=106—109°C — Rendement: 71%.
Chlorhydrate, F: 187°C — Rendement: 100%.

Exemple 10

(N-n propylaminométhyl)-6 tétrahydro-4,5,6,7 benzo[d]thiazole et son chlorhydrate
(I, R=H, R'=$nC_3H_7$, substitution en 6)

Il est préparé à partir du tosylate (V) décrit dans l'exemple 8, par action de la n-propylamine en solution dans le méthanol, en autoclave à 90°C. $eb_{0,05}$=96—100°C — Rendement: 61%.
La base est transformée en chlorhydrate par HCl en solution dans l'éther anhydre. F=166°C — Rendement: 87%.

Exemple 11

n-Butyl-2 (N-méthylaminométhyl)-4 tétrahydro-4,5,6,7 benzo[d]thiazole et son chlorhydrate
(I, R=$nC_4H_9$, R'=$CH_3$, substitution en 4)

1) n-Butyl-2 éthoxycarbonyl-4 tétrahydro-4,5,6,7 benzo[d]thiazole (III)

Il est obtenu par action du thiovaléramide sur la bromo-2 éthoxycarbonyl-6 cyclohexanone selon le procédé décrit dans l'exemple 1. $eb_{0,5}$=116—128°C — Rendement: 28%.

2) n-Butyl-2 hydroxyméthyl-4 tétrahydro-4,5,6,7 benzo[d]thiazole (IV)

Il est préparé par réduction du dérivé (III) précédent, par $AlLiH_4$ dans l'éther à température ordinaire. $eb_{0,6} = 130-132°C$ — Rendement: 48%.

3) n-Butyl-2 tosyloxyméthyl-4 tétrahydro-4,5,6,7 benzo[d]thiazole (V)

Il est préparé par action du chlorure de p. toluène sulfonyle sur le dérivé (IV) précédent, selon le procédé décrit dans l'exemple 1. Huile indistillable, utilisée brute pour la suite des réactions.

4) n-Butyl-2 (N-méthylaminométhyl)-4 tétrahydro-4,5,6,7 benzo[d]thiazole et son chlorhydrate
($I$, $R = nC_4H_9$, $R' = CH_3$)

Il est préparé par action de la méthylamine en solution méthanolique sur le tosylate (V) précédent, en autoclave à 90°C. $eb_{0,5} = 118-120°C$ — Rendement: 74%.
La base est transformée en chlorhydrate par HCl dans l'éther anhydre.
F: 128°C — Rendement: 70%.

Exemple 12

n-Butyl-2 (N-méthylaminométhyl)-5 tétrahydro-4,5,6,7 benzo[d]thiazole et son chlorhydrate
($I$, $R = nC_4H_9$, $R' = CH_3$, substitution en 5)

1) n-Butyl-2 éthoxycarbonyl-5 tétrahydro-4,5,6,7 benzo[d]thiazole (III)

Il est obtenu par action du thiovaléramide sur la bromo-2 éthoxycarbonyl-5 cyclohexanone dans le diméthylformamide, à température ordinaire. $eb_{0,5} = 140-142°C$ — Rendement: 56%.

2) n.Butyl-2 hydroxyméthyl-5 tétrahydro-4,5,6,7 benzo[d]thiazole (IV)

Il est préparé par réduction du dérivé (III) précédent par $AlLiH_4$ dans l'éther à température ordinaire $eb_{0,15} = 138-142°C$ — Rendement: 89%.

3) n.Butyl-2 tosyloxyméthyl-5 tétrahydro-4,5,6,7 benzo[d]thiazole (V)

Il est préparé par action du chlorure de p. toluène sulfonyle sur le dérivé IV précédent, selon le procédé décrit dans l'exemple 1. Huille indistillable utilisée brute pour la suite des réactions.

4) n.Butyl-2 (N-méthylaminométhyl)-5 tétrahydro-4,5,6,7 benzo[d]thiazole et son chlorhydrate
($I$, $R = nC_4H_9$), $R' : CH_3$)

Il est préparé par action de la méthylamine en solution méthanolique sur le tosylate (V) précédent, en autoclave à 90°C. $eb_{0,15} = 122-124°C$ — Rendement: 72%.
La base est transformée en chlorhydrate par HCl dans l'éther anhydre. F = 169–171°C — Rendt: 100%.

Exemple 13

Isopropyl-2 (N-méthylaminométhyl)-5 tétrahydro-4,5,6,7 benzo[d]thiazole et son chlorhydrate
($I$, $R$: $iC_3H_7$, $R'$: $CH_3$, substitution en 5)

1) Isopropyl-2 éthoxycarbonyl-5 tétrahydro-4,5,6,7 benzo[d]thiazole (III)

Il est obtenu par action du thio-isobutyramide sur la bromo-2 éthoxycarbonyl-5 cyclohexanone, dans le diméthylformamide, à température ordinaire. $eb_{0,05} = 109-111°C$ — Rendement: 50%.

2) Isopropyl-2 hydroxyméthyl-5 tétrahydro-4,5,6,7 benzo[d]thiazole (IV)

Il est préparé par réduction du dérivé (III) précédent par $AlLiH_4$ dans l'éther à température ordinaire. $eb_{0,1}$ = 122–126° C — Rendement: 83%.

3) Isopropyl-2 tosyloxymethyl-5 tétrahydro-4,5,6,7 benzo[d]thiazole (V)

Il est préparé par action du chlorure de p. toluène sulfonyl sur le dérivé (IV) précédent, selon le procédé décrit dans l'exemple 1. F = 82–83° C — Rdt 71%.

4) Isopropyl-2 (N-méthylaminométhyl)-5 tétrahydro-4,5,6,7 benzo[d]thiazole et son chlorhydrate (I, R: $iC_3H_7$, R' = $CH_3$)

Il est préparé par action de la méthylamine en solution méthanolique sur le tosylate (V) précédent, en autoclave à 90° C. $eb_{0,2}$ = 108–109° C — Rendement: 85%.

La base est transformée en chlorhydrate par HCl dans l'éther anhydre. F: 177° C — Rendement: 98%.

On a rassemblé dans le tableau I suivant d'autres exemples de composés de formule I préparés de manière analogue.

Tableau I

| Ex. | Formule | Désignation | Caractéristiques physiques | Rendt % |
|---|---|---|---|---|
| 14 | 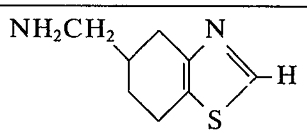 | aminométhyl-5 tétrahydro-4,5,6,7 benzo[d]thiazole | base $eb_{0,7}$ = 116-118°C<br>chlorhydr. F = 119-201°C | 68<br>95 |
| 15 | 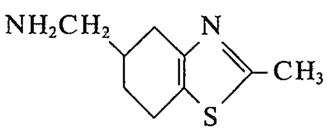 | méthyl-2 amino-méthyl-5 tétra-hydro-4,5,6,7 benzo[d]thiazole | base $eb_{0,5}$ = 115-125°C<br>chlorhydr. F = 262-263°C | 48<br>100 |
| 16 | 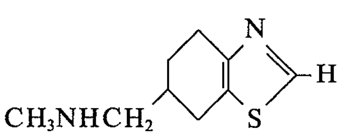 | (N-méthylamino-méthyl)-6 tétra-hydro-4,5,6,7 benzo[d]thiazole | base $eb_{0,3}$ = 98-102°C<br>chlorhydr. F = 225-227°C | 51<br>77 |
| 17 | 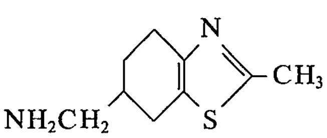 | méthyl-2 amino-méthyl-6 tétra-hydro-4,5,6,7 benzo[d]thiazole | base $eb_{0,5}$ = 120-130°C<br>chlorhydr. F = 206-207°C | 70<br>72 |
| 18 | 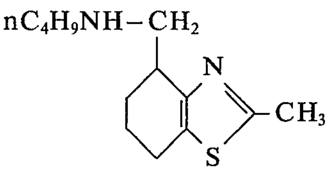 | méthyl-2 (N-n-butylamino-méthyl)-4 tétra-hydro-4,5,6,7 benzo[d]thiazole | base $eb_{0,6}$ = 118-122°C<br>chlorhydr. F = 132°C | 76<br>94 |
| 19 | 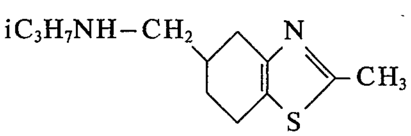 | méthyl-2 (N-iso-propylamino-méthyl)-5 tétra-hydro-4,5,6,7 benzo[d]thiazole | base $eb_{0,2}$ = 102-106°C<br>chlorhydr. F = 204-205°C | 82<br>100 |
| 20 | 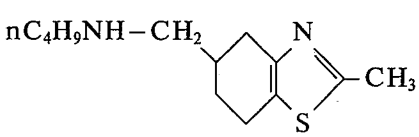 | méthyl-2 (N-n-butylamino-méthyl)-5 tétra-hydro-4,5,6,7 benzo[d]thiazole | base $eb_{0,1}$ = 112-118°C<br>chlorhydr. F = 245°C | 81<br>100 |

Les composés de formule I et leurs sels d'addition pharmaceutiquement acceptables possèdent des propriétés pharmacologiques intéressantes, particulièrement sur le système cardiovasculaire. Ainsi, ils augmentent, notablement pour certains d'entre eux, le débit sanguin de perfusion des membres.

Cette activité sur le système cardiovasculaire a été mise en évidence dans un test classique chez le chien, où on a mesuré l'effet sur le débit sanguin artériel fémoral. Ainsi, on a observé pour certains d'entre eux une augmentation du débit artériel dès la dose de 0,1 à 0,5 mg/kg I.V.

En outre, on a observé, lors d'une occlusion aigüe de l'artère fémorale, une nette diminution de la résistance des artères collatérales avec les composés des exemples 6 et 11, sans que soient notablement affectées la tension artérielle systémique, ni la pression de perfusion tissulaire.

Dans leur majorité, les composés de formule I sont peu toxiques, comme cela apparaît à la lecture du tableau suivant, où sont rassemblées les $DL_{50}$ par voie orale et par voie intrapéritonéale chez la souris.

Tableau II

Toxicité aigüe chez la souris

| Ex. | Voie orale (mg/kg) | Voie intrapéritonéale (mg/kg) |
|---|---|---|
| 1 | $DL_{50} > 200$ | $DL_{50} = 140$ |
| 2 | $DL_{50} > 200$ | $DL_{50} \geqslant 200$ |
| 3 | $DL_{50} > 200$ | – |
| 5 | $DL_{50} > 200$ | $DL_{50} > 200$ |
| 6 | $DL_{50} > 200$ | – |
| 7 | $DL_{50} > 200$ | $DL_{50} > 200$ |
| 8 | $DL_{50} > 200$ | $DL_{50} > 200$ |
| 9 | $DL_{50} > 200$ | $DL_{50} = 120$ |
| 10 | $DL_{50} > 200$ | $DL_{50} = 190$ |
| 11 | $DL_{50} \geqslant 200$ | $DL_{50} = 58$ |
| 12 | $DL_{50} > 200$ | $DL_{50} = 140$ |
| 13 | $DL_{50} > 200$ | $DL_{50} = 140$ |
| 14 | $DL_{50} \geqslant 200$ | $DL_{50} > 200$ |
| 15 | $DL_{50} > 200$ | $DL_{50} > 200$ |
| 16 | $DL_{50} = 170$ | $DL_{50} = 75$ |
| 17 | $DL_{50} > 200$ | $DL_{50} > 200$ |
| 18 | $DL_{50} > 200$ | $DL_{50} = 58$ |
| 19 | $DL_{50} > 200$ | $DL_{50} > 200$ |
| 20 | $DL_{50} = 145$ | $DL_{50} = 58$ |

Les composés de formule I et leurs sels pharmaceutiquement acceptables peuvent être utilisés en thérapeutique dans le traitement des insuffisances circulatoires de différentes origines.

Les composés des exemples 6 et 11 paraissent à cet égard particulièrement intéressants.

Les composés sont administrables à l'homme par voie orale, rectale ou parentérale, à l'état de base ou de sels, en solution aqueuse ou autre, en suspension, ou sous forme cristalline. Les différentes présentations peuvent contenir de 10 à 500 mg de principe actif par unité de prise et la posologie journalière chez l'adulte peut varier de 20 mg à 2 g selon les indications thérapeutiques envisagées.

**Revendications**

1. Composés de formule générale

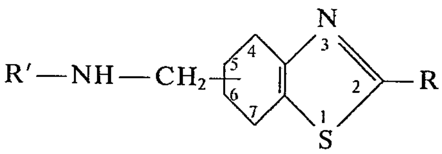

(I)

dans laquelle R représente un atome d'hydrogène ou un radical alcoyle en $C_1$ à $C_6$,
R' représente un atome d'hydrogène ou un radical alcoyle en $C_1$ à $C_6$,
ainsi que leurs sels d'addition avec des acides pharmaceutiquement acceptables.

2. Le méthyl-2 (N-méthylaminométhyl)-5 tétrahydro-4,5,6,7 benzo[d]thiazole, et ses sels pharmaceutiquement acceptables.

3. Le n-butyl-2 (N-méthylaminométhyl)-4 tétrahydro-4,5,6,7 benzo[d]thiazole, et ses sels pharmaceutiquement acceptables.

4. Procédé de préparation de composés de formule I telle que définie à la revendication, et de leurs sels d'addition pharmaceutiquement acceptables, caractérisé en ce que

a) on fait réagir un céto-ester bromé de formule

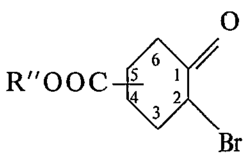

(II)

dans laquelle R″ est un radical alcoyle en $C_1 - C_2$, un thioamide de formule

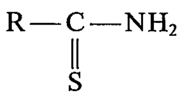

dans laquelle R a la signification donnée précédemment, obtenant ainsi un ester de formule

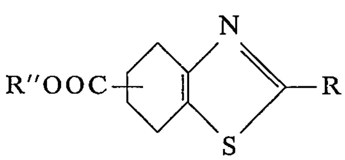

(III)

b) on réduit l'ester de formule III à l'aide d'aluminohydrure de lithium en un alcool de formule

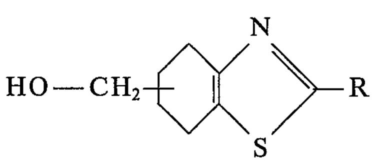

(IV)

c) on transforme l'alcool de formule IV au moyen de chlorure de p-toluènesulfonyle en le tosylate correspondant de formule

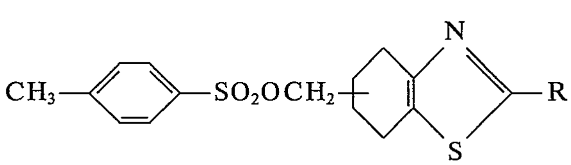

(V)

d) on fait réagir sur le tosylate de formule V une amine de formule $R'-NH_2$, R' ayant la signification donnée ci-dessus de façon à obtenir une amine de formule I, et éventuellement

e) on transforme l'amine de formule I en un sel avec un acide pharmaceutiquement acceptable.

5. Composition thérapeutique, caractérisée en ce qu'elle contient à titre de principe actif un composé de formule I ou l'un de ses sels d'addition avec un acide pharmaceutiquement acceptable.

**Patentansprüche**

1. Verbindungen der allgemeinen Formel

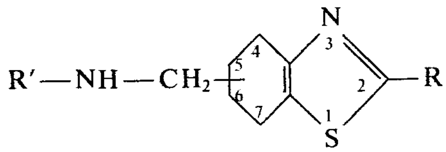

(I)

in der R ein Wasserstoffatom oder ein $C_1-C_6$-Alkylrest, R' ein Wasserstoffatom oder ein $C_1-C_6$-Alkylrest ist, sowie ihre Additionssalze mit pharmazeutisch unbedenklichen Säuren.

2. 2-Methyl-5-(N-methylaminoethyl)-4,5,6,7-tetrahydrobenzo[d]thiazol und seine pharmazeutisch unbedenklichen Salze.

3. 2-n-Butyl-4-(N-methylaminomethyl)-4,5,6,7-tetrahydrobenzo[d]thiazol und seine pharmazeutisch unbedenklichen Salze.

4. Verfahren zur Herstellung von Verbindungen der Formel I, wie sie in Anspruch 1 definiert ist, und ihrer pharmazeutisch unbedenklichen Additionssalze, dadurch gekennzeichnet, daß

a) man einen bromierten Ketoester der Formel

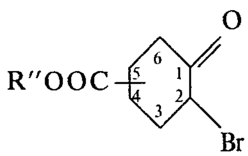

(II)

in der R" ein $C_1-C_2$-Alkylrest ist, und ein Thioamid der Formel

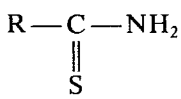

in der R die vorstehend genannte Bedeutung hat, umsetzt, wobei man in dieser Weise einen Ester der Formel

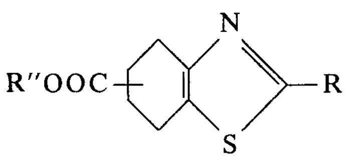

(III)

erhält,

b) man den Ester der Formel III mit Hilfe von Lithiumaluminohydrid zu einem Alkohol der Formel

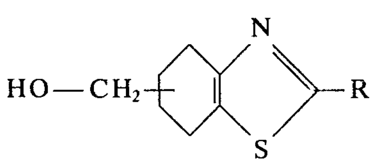

(IV)

reduziert,

c) man den Alkohol der Formel IV mit Hilfe von p-Toluolsulfonylchlorid in das der Formel

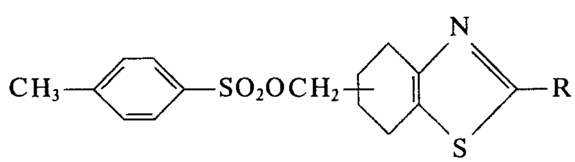

(V)

entsprechende Tosylat umwandelt,

d) man mit dem Tosylat der Formel V ein Amin der Formel R'−$NH_2$, in der R' die vorstehend genannte Bedeutung hat, in einer solchen Weise umsetzt, daß man ein Amin der Formel I erhält, und gegebenenfalls

e) das Amin der Formel I in ein Salz mit einer pharmazeutisch unbedenklichen Säure umwandelt.

5. Arzneimittelzubereitung, dadurch gekennzeichnet, daß sie als Wirkstoff eine Verbindung der Formel I oder eines ihrer Additionssalze mit einer pharmazeutisch unbedenklichen Säure enthält.

**Claims**

1. Compounds of the general formula (I):

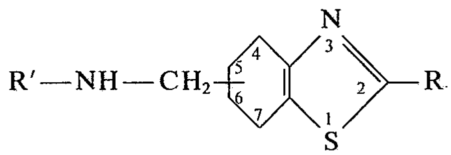

(I)

in which:

R is selected from hydrogen and $C_{1-6}$ alkyl,
R' is selected from hydrogen and $C_{1-6}$ alkyl;

and their pharmaceutically acceptable acid addition salts.

2. 2-Methyl-5-(N-methylaminomethyl)-4,5,6,7-tetrahydrobenzo[d]thiazole and its pharmaceutically acceptable acid addition salts.

3. 2-n-Butyl-4-(N-methylaminomethyl)-4,5,6,7-tetrahydrobenzo[d]thiazole and its pharmaceutically acceptable acid addition salts.

4. Process for the preparation of compounds of the formula (I) as defined in claim 1 and their pharmaceutically acceptable acid addition salts, comprising:

a) reacting a bromo keto-ester of the formula (II):

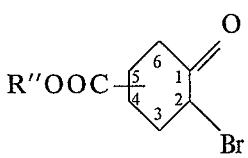

(II)

in which R″ is selected from $C_{1-2}$ alkyl and a thioamide of the formula

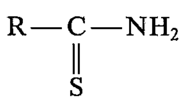

in which R is as defined in claim 1, to give an ester of the formula (III):

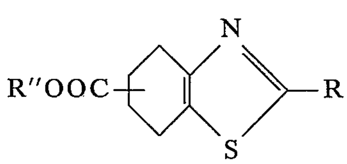

(III)

in which R″ has the above-defined meaning;

b) reducing the ester of the formula (III) with lithium aluminum hydride to give an alcohol having the formula (IV):

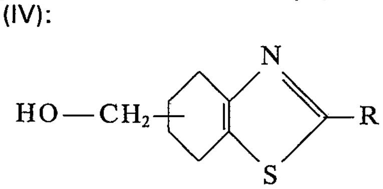

(IV)

in which R is as defined in claim 1;

c) converting the alcohol of the formula (IV), with p-toluenesulfonyl chloride, to the corresponding tosylate having the formula (V):

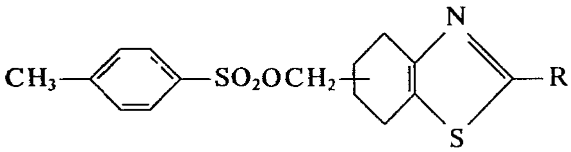

(V)

d) reacting the tosylate of the formula (V) with an amine of the formula $R'-NH_2$ in which R' is as defined in claim 1, to give an amine of the formula (I), and, if desired,

e) converting the amine of the formula (I) to a pharmaceutically acceptable acid addition salt.

5. Therapeutic composition, comprising, as active ingredient, a compound of the formula (I) or a pharmaceutically acceptable acid addition salt thereof.